# EUROPÄISCHE PATENTANMELDUNG

(11) **EP 3 069 741 A1**
(43) Veröffentlichungstag der Anmeldung: **21.09.2016**
(21) Anmeldenummer: 15159496.7
(22) Anmeldetag: 17.03.2015
(51) Int. Cl.: A61M 1/12, A61M 1/10

(54) **Herzpumpeneinrichtung und Verfahren zu ihrem Betrieb**

(71) Anmelder: Berlin Heart GmbH, 12247 Berlin (DE)
(72) Erfinder: Kallenbach, Sebastian, 13353 Berlin (DE); Krambeck, Helge, 12163 Berlin (DE); Breuel, Dr., Ralph, 12167 Berlin (DE)
(74) Vertreter: Pfenning, Meinig & Partner mbB

(57) **Zusammenfassung**

Die Erfindung bezieht sich auf eine Herzpumpeneinrichtung mit einer implantierbaren Herzpumpe (1, 1'), die wenigstens einen Sensor (18, 19, 20, 21, 22) aufweist, wobei unter den Sensoren zumindest ein Sensor (22) für einen Rotor (7, 7') der Herzpumpe ist, und mit einer Steuereinrichtung (26), die mit der Herzpumpe (1, 1') mittels einer transkutanen Leitung (25) verbunden ist, gekennzeichnet durch eine Signalverarbeitungseinrichtung (23, 24, 24', 31, 32), die einerseits mittels der transkutanen Leitung (25) mit der Steuereinrichtung (26) verbunden ist und die andererseits mit wenigstens einem Sensor (18, 19, 20, 21, 22) der Herzpumpe verbunden ist und Signale wenigstens eines Sensors über die transkutane Leitung (25) an die Steuereinheit (26) übermittelt. Die Signalverarbeitungseinrichtung ist dabei für eine Vorverarbeitung der Sensordaten zur effizienteren Übertragung über die transkutane Leitung vorgesehen.

## Beschreibung

Die Erfindung liegt auf dem Gebiet der Elektrotechnik und speziell der Medizintechnik und ist konkret im Zusammenhang mit Herzpumpen einsetzbar.

Seit einiger Zeit sind zur Unterstützung des menschlichen Herzens implantierbare elektrische Herzpumpen bekannt, die beispielsweise als LVAD (left ventricular assist device), RVAD (right ventricular assist device) oder BiVAD (bi-ventricular assist device) ausgeführt sein können und in diesem Fall Blut vom linken Herzventrikel in die Aorta befördern. Derartige Pumpen werden üblicherweise von außerhalb des Körpers mit Energie versorgt und auch gesteuert. Hierzu sind üblicherweise jeweils ein oder mehrere drahtgebundene Leitungen vorgesehen, die die eigentliche Herzpumpe, insbesondere einen in der Herzpumpe vorgesehenen Motor, mit elektrischer Energie versorgen und ansteuern. Zudem werden innerhalb der implantierten Herzpumpe über Sensoren Daten erfasst, wie beispielsweise die Rotorposition der Herzpumpe, die Pumpentemperatur, der Ventrikelblutdruck, die Bluttemperatur, Lageinformationen, Winkelbeschleunigungen, akustische Signale, der intrathorakale Druck und ähnliche Größen, und diese werden durch eine außerhalb des Patientenkörpers befindliche Steuereinrichtung empfangen und bearbeitet, insbesondere auch gespeichert.

Üblicherweise ist zur Verbindung der Sensoren und des Pumpenmotors mit der Steuereinrichtung eine Anzahl von Leitungen bzw. Adern von Leitungen vorgesehen, um alle Daten sowie die Energie in Form einer Einzelverdrahtung durch ein kombiniertes transkutanes bzw. perkutanes Kabel zu transportieren. Aufgrund der großen Anzahl von Adern ist der Querschnitt der transkutanen Leitungen bzw. des Kabels relativ groß, so dass die Hautdurchtrittsstelle ein erhöhtes Infektionsrisiko bildet. Zudem wird eine derartige kombinierte Leitung durch einen großen Querschnitt steif und bruchanfällig. Die Zahl der Sensoren ist hierdurch begrenzt, da durch weitere hinzuzufügende Leitungen der Durchmesser der kombinierten Leitung weiter wächst.

Es sind auch Lösungen bekannt, bei denen eine Übertragung von Daten über eine Funkverbindung von innerhalb des Körpers zum Körperäußeren statt-findet, jedoch hat eine derartige Kommunikationsverbindung gewisse Ausfallrisiken, so dass als Rückfallposition eine drahtgebundene Verbindung wünschenswert ist. Beispielsweise kann eine laufend ermittelte Rotorposition des Antriebs der Herzpumpe für die Antriebssteuerung wichtig sein.

Vor dem Hintergrund des Standes der Technik liegt somit der vorliegenden Erfindung die Aufgabe zugrunde, eine körperexterne Steuereinrichtung für eine Herzpumpeneinrichtung an eine Herzpumpe mit möglichst geringem Aufwand und minimiertem Infektionsrisiko anzubinden.

Die Aufgabe wird mit den Merkmalen der Erfindung gemäß Patentanspruch 1 gelöst. Die Unteransprüche 2 bis 11 stellen vorteilhafte Ausgestaltungen der Erfindung dar.

Die Erfindung bezieht sich somit auf eine Herzpumpeneinrichtung mit einer implantierbaren Herzpumpe, die wenigstens einen Sensor aufweist, wobei unter den Sensoren zumindest einer der nachfolgenden Sensoren ist: ein Positionssensor für einen Rotor der Herzpumpe; ein Lage- und/oder Beschleunigungssensor; ein Volumenstromsensor; ein Temperatursensor; ein Drucksensor; ein Druckdifferenzsensor; ein Sauerstoffsättigungssensor; ein chemischer Sensor zur Blutanalyse. Die Herzpumpeneinrichtung umfasst ferner eine Steuereinrichtung, die mit der Herzpumpe mittels einer transkutanen oder perkutanen Leitung verbunden ist. Unter einer transkutanen Leitung wird beispielsweise eine durch die Haut führende Leitung verstanden. Im Gegensatz dazu verläuft eine perkutane Leitung beispielsweise gänzlich im Körper und wird oftmals zumindest an einer Stelle in die Nähe der Hautoberfläche geführt und beispielsweise mit einem TET(transcutaneous energy transfer)-System gekoppelt. Das TET-System wird hierbei derart konfiguriert, dass neben Energie auch vorzugsweise digitale Daten ausgetauscht werden können.

Weiter bezieht sich die Erfindung auf eine Signalverarbeitungseinrichtung, die einerseits mittels der transkutanen oder perkutanen Leitung mit der Steuereinrichtung verbunden ist und die andererseits mit wenigstens einem Sensor der Herzpumpe verbunden ist und in einem ersten Betriebszustand Signale des wenigstens einen Sensors an die Steuereinheit übermittelt. Dabei findet die Übermittlung der Signale in einigen Ausführungsformen über die transkutane bzw. perkutane Leitung statt.

Die Signalverarbeitungseinrichtung ist ebenso wie die Herzpumpe implantierbar und mit einem oder mehreren Sensoren der Herzpumpe unmittelbar verbunden und leistet eine Vorverarbeitung der Signale von einem oder mehreren Sensoren derart, dass diese Signale beispielsweise über die transkutane oder perkutane Leitung vereinfacht übermittelt werden können. Es können dabei auch Signale verschiedener Sensoren kombiniert oder verbunden werden, indem beispielsweise mehrere Signale über ein Bussystem zusammengefasst werden. Insbesondere kann die Signalverarbeitungseinrichtung in einem ersten Betriebszustand der Herzpumpeneinrichtung eine Vielzahl von zu übertragenden Signalen - oder in einigen Ausführungsbeispielen sämtliche zu übertragende Signale - von Sensoren kombinieren und über eine als Datenbus ausgeführte transkutane bzw. perkutane Leitung übermitteln. Die transkutane Leitung kann zu diesem Zweck eine oder mehrere Adern von elektrischen Leitern aufweisen und als Leitung für einen seriellen oder parallelen Bus dienen. Es ist auch eine Übermittlung über eine optische Signalleitung durch die Signalverarbeitungseinrichtung möglich. Außerdem ist eine Kombination der energieübertragenden Leitungen mit den datenübertragenden Leitungen möglich (Power-Line-Kommunikation).

Üblicherweise werden zur Einrichtung eines Datenbussystems weniger Leitungen benötigt als bei einer Einzelverdrahtung der Sensoren und einzelner Verbindung der Sensoren zu der außerhalb des Patientenkörpers angeordneten Steuereinrichtung.

Zudem kann die Signalverarbeitungseinrichtung Daten der einzelnen Sensoren speichern und beispielsweise auch für eine Eichung der Signale entsprechende Daten speichern. Dies hat den Vorteil, dass die Daten unabhängig von dem weiteren Kommunikationsweg, den sie zwischen der Signalverarbeitungseinrichtung und der Steuereinrichtung zurücklegen, in der Steuereinrichtung interpretiert werden können. Die bei der Steuereinrichtung eintreffenden Informationen sind nicht von der einzelnen Paarung zwischen einer Herzpumpe und einer Steuereinrichtung abhängig. Bei einem Tausch der Steuereinrichtung muss somit keine Eichung oder Initialisierung mit einer konkreten Herzpumpe erfolgen. Dies führt zu starken Vereinfachungen bei der Lagerhaltung und Installation von Steuereinrichtungen. Es entfällt auch die Notwendigkeit, jeweils Herzpumpe und Steuereinrichtung als festes Paar einzurichten und einzusetzen.

Die Signalverarbeitungseinrichtung ist in einigen Ausführungsbeispielen zumindest mit einem der vorstehend aufgeführten Sensoren verbunden. In einer Ausführungsform ist wenigstens einer der Sensoren ein Positionssensor eines Rotors der Herzpumpe. In diesem Falle ist der Positionssensor beispielsweise ein Sensor, der die Position des rotierenden Rotors eines elektrischen Motors in der Herzpumpe darstellt. Die Übermittlung derartiger Positionsdaten ist für die Ansteuerung insbesondere von bürstenlosen Elektromotoren wichtig, die in der Medizintechnik mit Vorteil eingesetzt werden, da sie extrem effizient, gut ansteuerbar und wartungsarm sind.

Als Sensor für die Position des Rotors der Herzpumpe kann auch ein Sensor dienen, der durch Ermittlung von Strom und Spannung eines bürstenlosen Elektromotors auf rechnerischem Weg über die Läufer-Rückwirkung die Winkelposition des Läufers/Rotors ermittelt.

Die Signalverarbeitungseinrichtung kann zudem auch mit weiteren Sensoren, beispielsweise zwei, drei, vier oder mehr Sensoren, verbunden sein, wobei diese Sensoren in oder an der Herzpumpe vorgesehen sein können und beispielsweise als einer der vorstehenden Sensoren oder als Pumpentemperatursensor, Blutdrucksensor oder Bluttemperatursensor ausgebildet sind. Die Signalverarbeitungseinrichtung und die implantierbare Herzpumpe können mitsamt den verwendeten Sensoren bei der Inbetriebnahme oder werksseitig vor einer Inbetriebnahme eingemessen werden, wobei über bekannte Referenzwerte eine Eichung erfolgt und entsprechende Eichparameter in der Signalverarbeitungseinrichtung gespeichert werden können. Damit kann die Herzpumpe gemeinsam mit der Signalverarbeitungseinrichtung mit jeder externen Steuereinrichtung ohne weitere Eichung zusammenarbeiten. Die externe Steuereinrichtung ist damit ohne Probleme ersetzbar und ohne weitere Einmessung einsetzbar.

Vorteilhaft ist die Signalverarbeitungseinrichtung unmittelbar an der Herzpumpe angeordnet. Dies erlaubt eine problemlose gemeinsame Implantierung von Herzpumpe und Signalverarbeitungseinrichtung und zudem kurze Signalwege zwischen den Sensoren der Herzpumpe und der Signalverarbeitungseinrichtung. Die Signalverarbeitungseinrichtung kann auch mechanisch an der Herzpumpe gehalten, beispielsweise an diese anmontiert sein, so dass hierdurch die relative Position von Herzpumpe und Signalverarbeitungseinrichtung eindeutig festgelegt ist.

Eine weitere vorteilhafte Ausgestaltung der Erfindung sieht vor, dass eine Sendeeinrichtung in unmittelbarer Nähe der Herzpumpe vorgesehen, insbesondere unmittelbar mit dieser verbunden ist und die Signale wenigstens eines Sensors verarbeitet und mittels einer drahtlosen Verbindung versendet. Für den Normalfall eines störungsfreien Betriebs können somit die von den Sensoren erfassten Daten ohne Verwendung der transkutanen Leitung über Funk übertragen werden. Es ist jedoch denkbar, beide Übertragungswege parallel zu verwenden und die übertragenen Signale in der Steuereinrichtung zu vergleichen, um eine höhere Zuverlässigkeit zu erreichen. Wenigstens in dem Fall, dass die Sendeeinrichtung ausfällt, wird unmittelbar auf die Signalverarbeitungseinrichtung und die Übermittlung über die transkutane Leitung umgeschaltet. Zudem wird die transkutane bzw. perkutane Leitung dauerhaft zur Übertragung elektrischer Energie in Form einer Speisespannung eines Motors der Herzpumpe benötigt.

Eine weitere vorteilhafte Ausführung der Erfindung sieht vor, dass innerhalb der Signalverarbeitungseinrichtung ein Schaltmodul vorgesehen ist, das bewirkt, dass im Fall eines Fehlers der Signalverarbeitungseinrichtung ein zweiter Betriebszustand ausgeführt wird, in welchem das Signal des Positionssensors (oder des jeweiligen verwendeten Sensors) unter Umgehung wesentlicher Teile der Signalverarbeitungseinrichtung unmittelbar über die transkutane bzw. perkutane Leitung übertragen wird.

Wird beispielsweise ein Positionssensor für einen Rotor einer Herzpumpe verwendet und werden die mit diesem Positionssensor erfassten Daten im ersten Betriebszustand der Herzpumpeneinrichtung mittels der Signalverarbeitungseinrichtung verarbeitet und über die transkutane oder perkutane Leitung übertragen, so werden die Signale im zweiten Betriebszustand im Wesentlichen nicht durch die Signalverarbeitungseinrichtung verarbeitet, sondern direkt in die transkutane oder perkutane Leitung gespeist. Da eine laufende und zuverlässige Übermittlung der axialen Position des Rotors und/ oder der Winkelposition des Motors der Herzpumpe für den Betrieb und die Ansteuerung des Motors je nach verwendetem Pumpentyp sehr wichtig sein kann, müssen diese Größen des entsprechenden Sensors mit erhöhter Sicherheit übertragen werden. Aus diesem Grund kann die Signalverarbeitungseinrichtung eine Selbstüberwachungseinrichtung aufweisen, beispielsweise einen sogenannten Watchdog, der Fehlfunktionen detektiert und im Fall der Fehlfunktion mittels des Schaltmoduls den Signalweg, der den entsprechenden Sensor, insbesondere den Positionssensor, mit der Signalverarbeitungseinrichtung und weiter mit der Steuereinrichtung verbindet, umleitet, dergestalt, dass das Signal des Sensors an wesentlichen Teilen der Signalverarbeitungseinrichtung vorbei und unmittelbar über die transkutane bzw. perkutane Leitung zur Steuereinrichtung geleitet wird.

Es kann auch vorgesehen sein, dass weitere Sensoren derart qualifiziert sind, dass im Falle einer Fehlfunktion der Signalverarbeitungseinrichtung die von ihnen gelieferten Signale durch ein Schaltmodul unmittelbar und an wesentlichen Teilen der Signalverarbeitungseinrichtung vorbei über die transkutane bzw. perkutane Leitung zur Steuereinrichtung geleitet werden. Allerdings ist in diesem Fall, da die transkutane Leitung mit möglichst wenigen Adern auskommt, nicht notwendigerweise die Übermittlung aller Sensorsignale unmittelbar über die transkutane Leitung möglich. Allerdings ist die Übertragung der wichtigsten Signale über die transkutane bzw. perkutane Leitung im zweiten Betriebszustand auch dann sicher, falls die Signalverarbeitungseinrichtung beispielsweise ausfällt.

Zur vorteilhaften Ausgestaltung der Erfindung kann zudem beispielsweise vorgesehen sein, dass die Signalverarbeitungseinrichtung wenigstens eine Speichereinrichtung zur Speicherung von Systemparametern, insbesondere Parametern der Herzpumpe, und/oder von Messwerten eines oder mehrerer Sensoren aufweist. Eine derartige Speichereinrichtung lässt die Eichung der Paarung von Herzpumpe und Signalverarbeitungseinrichtung sowie die Speicherung kritischer Sensordaten zu, bei deren Eintreten oder Überschreiten beispielsweise Alarmsignale abgegeben werden sollen.

Vorteilhaft weist die Signalverarbeitungseinrichtung einen Mikrocontroller und/oder einen Transceiver, wie beispielsweise einen RS485-Transceiver, auf. Damit ist die Herzpumpeneinrichtung für eine effiziente Datenübermittlung mittels Kommunikationsstandards ausgerüstet, die möglichst wenig störanfällig sind.

Eine weitere vorteilhafte Ausgestaltung der Erfindung sieht vor, dass die Signalverarbeitungseinrichtung wenigstens eine flexible, insbesondere knickbare oder faltbare Leiterplatte aufweist. Die Signalverarbeitungseinrichtung ist üblicherweise als elektrische Schaltung mit einzelnen Schaltungselementen und/oder integrierten Schaltkreisen, beispielsweise auch ASICs, aufgebaut. Für den Aufbau eignen sich aufgrund des geringen Platzangebotes flexible oder faltbare Leiterplatinen, die entsprechend dem zur Verfügung stehenden Platzangebot angepasst werden können.

Besonders vorteilhaft kann in diesem Zusammenhang vorgesehen sein, dass die Signalverarbeitungseinrichtung ringförmig um einen Zu- oder Abflusskanal der Herzpumpe herum angeordnet ist. In Form einer knickbaren Leiterplatine kann die Signalverarbeitungseinrichtung ein ringförmiges Polygon bilden, das eine Zu- oder Abflussleitung der Pumpe umgibt.

Es kann auch vorgesehen sein, dass die Signalverarbeitungseinrichtung an einem ebenen Gehäuseboden der Herzpumpe angeordnet ist. Eine Leiterplatine der Signalverarbeitungseinrichtung kann somit einen Gehäuseboden der Pumpe bilden oder parallel zu einem Gehäuseboden der Pumpe verlaufen.

Außer auf eine Herzpumpeneinrichtung bezieht sich die vorliegende Erfindung auch auf ein Verfahren zum Betrieb einer Herzpumpeneinrichtung in einer der oben beschriebenen Ausführungsformen, bei dem die Herzpumpe mitsamt ihrer Signalverarbeitungseinrichtung mit einer Steuereinrichtung verbunden wird und bei dem Pumpenparameter erfasst und in der Signalverarbeitungseinrichtung gespeichert werden. Hieraus ergibt sich, dass die Pumpe gemeinsam mit der Signalverarbeitungseinrichtung eingemessen und gegebenenfalls geeicht wird und dass somit die Herzpumpe mit jeder Steuereinrichtung zusammenarbeitet, ohne dass eine weitere Anpassung notwendig wäre. Die notwendigen Eichparameter sind in der Speichereinrichtung der Signalverarbeitungseinrichtung abgelegt und abrufbar.

Im Betrieb leitet die Signalverarbeitungseinrichtung vorverarbeitete Signale von Sensoren über einen Datenbus mittels der transkutanen bzw. perkutanen Leitung weiter.

Im Folgenden wird die Erfindung anhand eines Ausführungsbeispiels in Figuren einer Zeichnung gezeigt und nachfolgend beschrieben. Dabei zeigt
- Fig. 1: einen Schnitt durch eine Herzwand mit einer implantierten Herzpumpe erster Bauart,
- Fig. 2: einen Schnitt durch eine Herzwand mit einer implantierten Herzpumpe zweiter Bauart,
- Fig. 3: eine dreidimensionale Außenansicht einer Herzpumpe mit einer im Wesentlichen polygonal ringförmigen Schaltplatine, die einen Einlassstutzen der Pumpe umgibt,
- Fig. 4: eine Ansicht eines Bodens einer Herzpumpe, in den eine Signalverarbeitungseinrichtung integriert ist,
- Fig. 5: die Zusammenführung der Sensordaten von verschiedenen Sensoren in einer Signalverarbeitungseinrichtung, die mittels eines Signalbusses mit einer Steuereinrichtung verbunden ist, sowie
- Fig. 6: eine Signalverarbeitungseinrichtung, die zumindest im Fehlerfall das Signal eines Positionssensors unter Umgehung wesentlicher Teile der Signalverarbeitungseinrichtung über die transkutane Leitung weiterleitet.

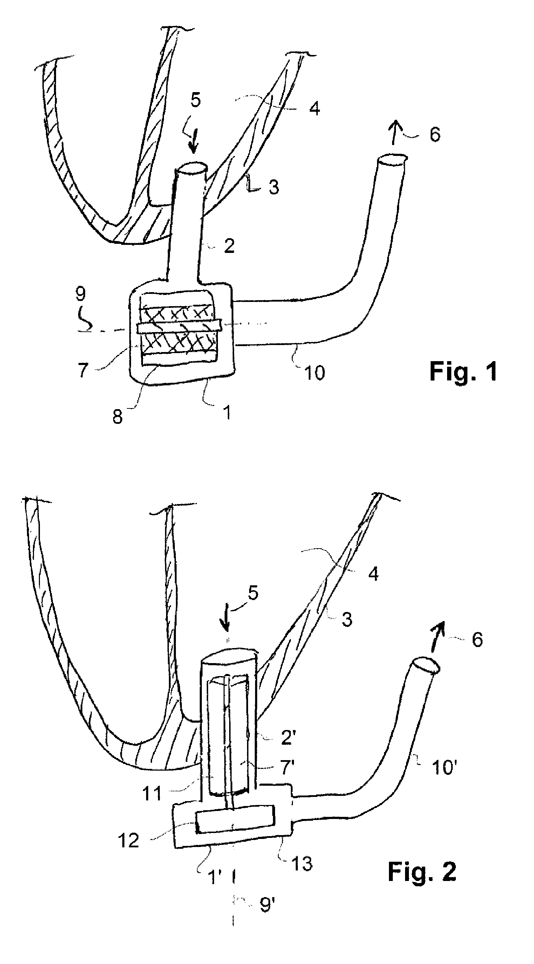

Figur 1 zeigt eine implantierbare Herzpumpe 1, deren Einlassstutzen 2 in eine Herzwand 3 im Bereich des linken Ventrikels eingelassen ist, derart, dass die Pumpe 1 aus der Herzkammer 4 in eine nicht dargestellte Arterie Blut fördern kann. Die Strömungsrichtung des Blutes ist durch die Pfeile 5, 6 angedeutet. Die Herzpumpe 1 weist einen Rotor 7 auf, der einerseits elektromagnetische Antriebselemente, wie einen Anker, sowie andererseits Förderelemente 8 in Form von Schaufeln aufweist, die Blut beispielsweise in Axialrichtung 9 zum Pumpenauslass 10 fördern. Im Bereich der Herzpumpe 1 sind üblicherweise Sensoren für verschiedene physiologische Größen, wie beispielsweise die Bluttemperatur und den Ventrikeldruck, angeordnet, sowie Sensoren für die Messung der Temperatur von Elementen der Pumpe, ein Sensor für die Beschleunigungsmessung, die über eine Bewegung des Patienten Aufschluss gibt, sowie ein Sensor, der die Winkelposition des Rotors der Pumpe erfasst.

Der Rotorpositionssensor ist insbesondere bei der Verwendung eines magnetischen Lagers notwendig, um die axiale Rotorposition zu bestimmen. Diese bildet eine wichtige Eingangsgröße für die Ansteuerung des Lagers und damit der magnetischen Lagerung des Rotors. Dies ist beispielsweise bei INCOR-Pumpen der Berlin Heart GmbH der Fall.

Figur 2 zeigt eine Herzpumpe 1', deren Einlassstutzen 2' ebenfalls in die Herzwand 3 im Bereich des linken Ventrikels 4 eingelassen ist, wobei die Rotationsachse 9' des Rotors 7' in Längsrichtung des Einlassstutzens 2' verläuft. Dabei sind der Antriebsteil 11 des Rotors mit dem Anker und gegebenenfalls auch einer Wicklung im Einlassstutzen 2' untergebracht, während der Rotorteil mit den Förderelementen 12 im Pumpengehäuse 13 angeordnet ist. Der Rotor 12 beschleunigt das Blut in radialer Richtung und teilweise in tangentialer Richtung, so dass es die Pumpe durch den Auslassstutzen 10' verlässt. Auch bei dieser Ausführungsform der Herzpumpe kann ein bürstenloser Elektromotor verwendet werden.

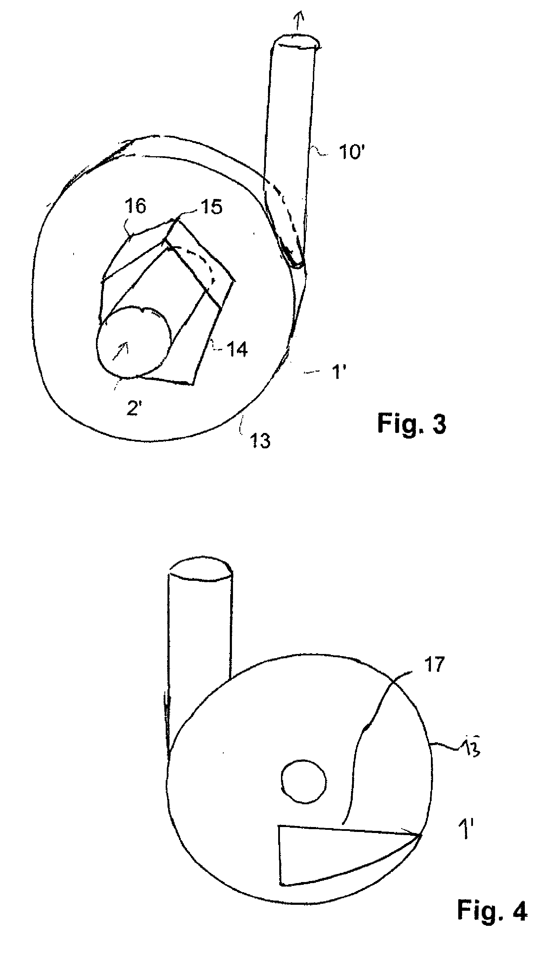

Figur 3 zeigt eine Außenansicht einer Herzpumpe 1' mit einem Einlassstutzen 2' und einem Auslassstutzen 10' sowie einem Pumpengehäuse 13 in annähernd zylindrischer Bauart, wobei um den Einlassstutzen 2' herum eine polygonal gefaltete Schaltplatine 14 dargestellt ist, die elektrische Bauteile einer Signalverarbeitungseinrichtung trägt, die jedoch im Einzelnen nicht dargestellt sind. Es soll in Figur 3 lediglich eine platzsparende Anordnung der Signalverarbeitungseinrichtung um den Einlassstutzen 2' herum zwischen dem Pumpengehäuse 13 und der Herzwand 3 gezeigt werden. Die Schaltplatine 14 kann an den Knickstellen 15, 16 beispielsweise Filmgelenkverbindungen zwischen einzelnen jeweils für sich flachen, ebenen Schaltplatinenteilen aufweisen, wobei Leiterbahnen der Schaltplatine die Knicklinien 15, 16 überschreiten können.

Figur 4 zeigt eine Ansicht eines Pumpengehäuses 13 vom Boden der Pumpe aus gesehen, wobei in den Boden ein Einlegeteil 17 eingebaut ist, das elektrische Bauelemente einer Signalverarbeitungseinrichtung tragen kann. Auch in dieser Weise kann eine Signalverarbeitungseinrichtung platzsparend und geschützt unmittelbar an der Herzpumpe 1' angeordnet werden.

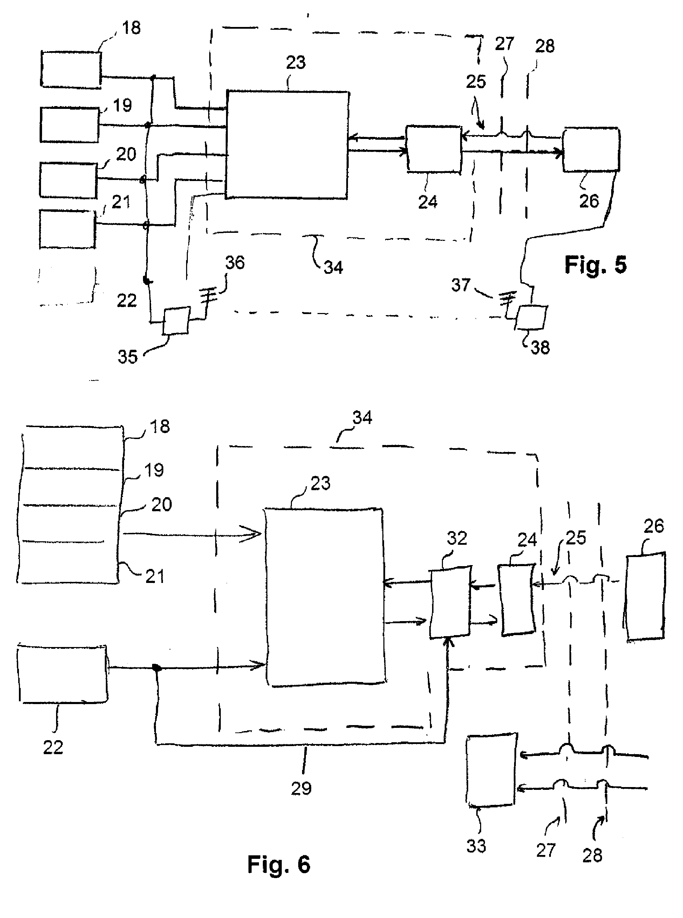

Figur 5 zeigt im Detail auf der linken Seite fünf Sensoren, nämlich einen Elektroniktemperatursensor 18, einen Beschleunigungssensor 19, einen Bluttemperatursensor 20, einen Ventrikeldrucksensor 21 sowie einen Rotorpositionssensor 22, von denen jeder mit einem Mikrocontroller 23 verbunden ist. Der Mikrocontroller 23 bildet zusammen mit einem Businterface 24 eine Signalverarbeitungseinrichtung 34, die über die transkutane bzw. perkutane Leitung 25 bidirektional mit einer Steuereinrichtung 26 kommuniziert. Dabei werden Sensormesswerte von den Sensoren 18, 19, 20, 21, 22 über die transkutane Busleitung 25 zur Steuereinrichtung 26 übertragen, und von der Steuereinrichtung 26 können Signale an die Herzpumpe, beispielsweise den Antrieb der Herzpumpe, geleitet werden. Dazu gehören beispielsweise elektrische Signale zur Ansteuerung des magnetischen Lagers. Die transkutane Leitung 25 kann zusätzlich zur Signalübertragung auch zur Übertragung elektrischer Energie genutzt werden.

Der Mikrocontroller 23 kann die Signale der Sensoren oder einer Auswahl der Sensoren zusammenfassen und derart aufbereiten, dass sie mittels eines gemeinsamen Kommunikationsprotokolls zur Steuereinrichtung 26 geschickt werden können. Hierdurch kann die notwendige Leitungskapazität der transkutanen Leitung 25, beispielsweise die Anzahl der benötigten Adern, auf ein Minimum reduziert werden, so dass die transkutane Leitung dünn und flexibel wird und dadurch weniger bruchanfällig ist und zudem eine geringe äußere Oberfläche aufweist und damit eine geringere Grenzfläche zum Gewebe des Patientenkörpers als potentielle Angriffsfläche für Infektionen bietet.

Durch die beiden gestrichelten Linien 27, 28 ist die Gewebeschicht angedeutet, durch die die transkutane Leitung 25 zum Körperäußeren hin verläuft, wo auch die Steuereinrichtung 26 angeordnet ist. Im Falle einer perkutanen Leitung würde diese beispielsweise in einem im Körper liegenden TET-Interface münden, welches mit einem außerhalb des Körpers angeordneten, korrespondierenden TET-Interface koppelbar ist. Eine Steuereinrichtung könnte im Körper, beispielsweise in der Nähe des innenliegenden TET-Interface und beabstandet von der Pumpe bzw. Pumpenelektronik, angeordnet sein. Alternativ kann die Steuereinrichtung auch außerhalb des Körpers angeordnet sein.

Parallel zu der transkutanen Leitung 25 ist eine Funkverbindung vorgesehen, mit einem implantierten Sender 35, einer Sendeantenne 36, einem Empfänger 38 und einer Empfangsantenne 37 außerhalb des Patientenkörpers.

In Figur 6 ist dargestellt, dass der Mikrocontroller 23 ausgehend von dem Rotorpositionssensor 22 durch eine Bypassleitung 29 umgehbar ist. Während im ersten Betriebszustand eine Vielzahl von Sensorsignalen durch die Signalverarbeitungseinrichtung verarbeitet wird, die Signalverarbeitungseinrichtung die Sensorsignale beispielsweise digitalisiert oder verschiedene Signale aggregiert, wird im zweiten Betriebszustand -beispielsweise im Falle einer Störung -die Vielzahl an Sensorsignalen oder in einigen Ausführungsbeispielen lediglich eine Teilmenge der Sensorsignale über die Bypassleitung 29 und unter Umgehung des Mikrocontrollers 23 zur außerhalb des Körpers liegenden Steuereinrichtung übertragen.

Üblicherweise werden die Sensordaten von den Sensoren 18, 19, 20, 21, 22 an den Mikrocontroller 23 geleitet, dort verarbeitet und vom Mikrocontroller 23 beispielsweise über einen RS485-Transceiver 24' zu der transkutanen Leitung 25 und über diese zu der externen Steuereinrichtung 26 geleitet. Das Signal des Rotorpositionssensors 22 oder ein die Position des Rotors widerspiegelndes Signal kann zusätzlich durch ein Tiefpassfilter geleitet werden, das dem Mikrocontroller vorgeschaltet ist.

In zahlreichen Ausführungsbeispielen ist eine Kontrolleinrichtung (beispielsweise ein Watchdog) vorgesehen, die die ordnungsgemäße Funktion des Mikrocontrollers 23 überprüft. Wird ein Fehler des Mikrocontrollers 23 signalisiert, so kann beispielsweise die Überwachungseinrichtung einen Schalter 32 betätigen, der beispielsweise ein Analogschalter ist und der die Herzpumpeneinrichtung von einem ersten Betriebszustand in einen zweiten Betriebszustand überführt, in welchem das Signal des Rotorpositionssensors 22, das ihn über die Bypassleitung 29 erreicht, zum Transceiver 24' durchgeschaltet wird. Damit wird das Signal des Rotorpositionssensors unmittelbar und ohne Passieren des Mikrocontrollers, also unter Umgehung wesentlicher Teile der Signalverarbeitungseinrichtung, an den Transceiver 24' und zur Steuereinrichtung 26 geleitet. Es ist dadurch mit hoher Sicherheit gewährleistet, dass die Signale, beispielsweise die Signale des Rotorpositionssensors, auch bei einer Fehlfunktion des Mikrocontrollers die Steuereinrichtung 26 erreichen, so dass auch der Antrieb der Herzpumpe unter Berücksichtigung der Rotorposition ordnungsgemäß gesteuert werden kann. Die Steuereinrichtung 26 ist zu diesem Zweck und zum Zweck der Energieübertragung entweder über eine zusätzliche, nicht dargestellte Leitung oder auch über die transkutane Leitung 25 mit dem Motorantrieb des Herzpumpenmotors verbunden.

Zudem ist in Figur 6 noch eine transkutane Verbindung vom Körperäußeren zu einer implantierten Spannungsversorgung 33 dargestellt. Die transkutane Verbindung kann jedoch alternativ auch perkutan ausgebildet sein. Im Falle einer perkutanen Leitung kann die oben genannte Verbindung ebenfalls perkutan oder transkutan ausgebildet sein.

Durch die Erfindung kann somit die transkutane Leitung 25 mit möglichst wenigen Adern und kleiner Oberfläche gewählt werden, wobei gleichzeitig eine hohe Funktionssicherheit der Herzpumpe sowie eine zuverlässige Ansteuerbarkeit gewährleistet ist.

## Patentansprüche

1. Herzpumpeneinrichtung mit einer implantierbaren Herzpumpe (1, 1'), die wenigstens einen Sensor (18, 19, 20, 21, 22) aufweist, wobei unter den Sensoren zumindest einer der nachfolgenden Sensoren ist:
- ein Positionssensor (22) für einen Rotor (7, 7') der Herzpumpe;
- ein Lage- und/oder Beschleunigungssensor;
- ein Volumenstromsensor;
- eine Temperatursensor;
- ein Drucksensor;
- ein Druckdifferenzsensor;
- ein Sauerstoffsättigungssensor;
- ein chemischer Sensor zur Blutanalyse;
und die Herzpumpeneinrichtung ferner eine Steuereinrichtung (26) umfasst, die mit der Herzpumpe (1, 1') mittels einer transkutanen oder perkutanen Leitung (25) verbunden ist, **gekennzeichnet durch** eine Signalverarbeitungseinrichtung (23, 24, 24', 31, 32), die einerseits mittels der transkutanen oder perkutanen Leitung (25) mit der Steuereinrichtung (26) verbunden ist und die andererseits mit wenigstens einem Sensor (18, 19, 20, 21, 22) der Herzpumpe verbunden ist und in einem ersten Betriebszustand Signale des wenigstens einen Sensors verarbeitet und an die Steuereinheit (26) übermittelt.

2. Herzpumpeneinrichtung nach Anspruch 1, **dadurch gekennzeichnet, dass** die Signalverarbeitungseinrichtung (23, 24, 24', 31, 32) unmittelbar an der Herzpumpe (1, 1') angeordnet ist.

3. Herzpumpeneinrichtung nach Anspruch 1 oder 2, **dadurch gekennzeichnet, dass** eine Sendeeinrichtung (35, 36) in unmittelbarer Nähe der Herzpumpe (1, 1') vorgesehen, insbesondere unmittelbar mit dieser verbunden ist, die Signale wenigstens eines Sensors (18, 19, 20, 21, 22) verarbeitet und mittels einer drahtlosen Verbindung (35, 36, 37, 38) versendet.

4. Herzpumpeneinrichtung nach Anspruch 1, 2 oder 3, **dadurch gekennzeichnet, dass** innerhalb der Signalverarbeitungseinrichtung (23, 24, 24', 31, 32) ein Schaltmodul (32) vorgesehen ist, das im Fall eines Fehlers der Signalverarbeitungseinrichtung das Signal des wenigstens einen Sensors in einem zweiten Betriebszustand unter Umgehung wesentlicher Teile der Signalverarbeitungseinrichtung unmittelbar über die transkutane Leitung (25) überträgt.

5. Herzpumpeneinrichtung nach Anspruch 1 oder einem der folgenden, **dadurch gekennzeichnet, dass** die Signalverarbeitungseinrichtung (23, 24, 24', 31, 32) wenigstens eine Speichereinrichtung zur Speicherung von Systemparametern (1, 1') und/oder von Messwerten eines oder mehrerer Sensoren (18, 19, 20, 21, 22) aufweist.

6. Herzpumpeneinrichtung nach Anspruch 1 oder einem der folgenden, **dadurch gekennzeichnet, dass** die Signalverarbeitungseinrichtung (23, 24, 24', 31, 32) einen Mikrocontroller (23) aufweist.

7. Herzpumpeneinrichtung nach Anspruch 1 oder einem der folgenden, **dadurch gekennzeichnet, dass** die Signalverarbeitungseinrichtung (23, 24, 24', 31, 32) einen Transceiver für eine digitale Buskommunikation aufweist.

8. Herzpumpeneinrichtung nach Anspruch 1 oder einem der folgenden, **dadurch gekennzeichnet, dass** unter anderem eine Versorgungsspannung über die transkutane oder perkutane Leitung (25) geleitet wird.

9. Herzpumpeneinrichtung nach Anspruch 1 oder einem der folgenden, **dadurch gekennzeichnet, dass** die Signalverarbeitungseinrichtung (23, 24, 24', 31, 32) wenigstens eine flexible, insbesondere knickbare oder faltbare Leiterplatte (14) aufweist.

10. Herzpumpeneinrichtung nach Anspruch 1 oder einem der folgenden, **dadurch gekennzeichnet, dass** die Signalverarbeitungseinrichtung (23, 24, 24', 31, 32) ringförmig um einen Zu- oder Abflusskanal (2, 2', 10, 10') der Herzpumpe (1, 1') herum angeordnet ist.

11. Herzpumpeneinrichtung nach einem der Ansprüche 1 bis 8, **dadurch gekennzeichnet, dass** die Signalverarbeitungseinrichtung (23, 24, 24', 31, 32) an einem ebenen Gehäuseboden der Herzpumpe (1, 1') angeordnet ist.

12. Verfahren zum Betrieb einer Herzpumpeneinrichtung nach einem der Ansprüche 1 bis 11, **dadurch gekennzeichnet, dass** die Herzpumpe (1, 1') mitsamt ihrer Signalverarbeitungseinrichtung (23, 24, 24', 31, 32) mit einer Steuereinrichtung (26) verbunden wird und dass Pumpenparameter erfasst und in der Signalverarbeitungseinrichtung gespeichert werden.

13. Verfahren gemäß Anspruch 12, **dadurch gekennzeichnet, dass** durch die Signalverarbeitungseinrichtung Signale von einem oder mehreren Sensoren (18, 19, 20, 21, 22) aufbereitet und mittels eines Kommunikationsprotokolls über einen Datenbus weitergeleitet werden.
